Europäisches Patentamt

(19) European Patent Office

Office européen des brevets

(11) EP 1 178 730 B1

(12) EUROPEAN PATENT SPECIFICATION

(45) Date of publication and mention
of the grant of the patent:
23.03.2005 Bulletin 2005/12

(51) Int Cl.⁷: A23C 9/123, A61P 37/02

(21) Application number: 00935083.6

(86) International application number:
PCT/EP2000/004593

(22) Date of filing: 05.05.2000

(87) International publication number:
WO 2000/067583 (16.11.2000 Gazette 2000/46)

(54) **USE OF LACTIC ACID BACTERIA IN THE PREPARATION OF FERMENTED MILKS FOR THE TREATMENT OF DEPRESSED IMMUNITY LEVELS**

VERWENDUNG VON MILCHSAÜREBAKTERIEN BEI DER HERSTELLUNG VON FERMENTIERTEN MILCHPRODUKTEN ZUR BEHANDLUNG VON VERRINGERTEN IMMUNITÄTSEBENEN

UTILISATION DE BACTERIES LACTIQUES DANS LA PREPARATION DE LAITS FERMENTES, POUR LE TRAITEMENT DE NIVEAUX D'IMMUNITE ALTERES

(84) Designated Contracting States:
AT BE CH CY DE DK ES FI FR GB GR IE IT LI LU MC NL PT SE

(30) Priority: 07.05.1999 ES 9901036
07.05.1999 ES 9901037

(43) Date of publication of application:
13.02.2002 Bulletin 2002/07

(73) Proprietor: Compagnie Gervais Danone
92302 Levallois Perret (FR)

(72) Inventors:
• PUJOL AMAT, Pere
E-08021 Barcelona (ES)
• POSTAIRE, Eric
F-92170 Vanves (FR)
• MATEOS GUARDIA, José, Antonio
E-08016 Barcelona (ES)

(74) Representative: Vialle-Presles, Marie José et al
Cabinet ORES,
36,rue de St Pétersbourg
75008 Paris (FR)

(56) References cited:
EP-A- 0 649 603       WO-A-98/23727
WO-A-99/10476

• OUWEHAND A C ET AL: "The health effects of cultured milk products with viable and non-viable bacteria." INTERNATIONAL DAIRY JOURNAL 8 (9) 749-758 1998 DEP. OF BIOCHEM. FOOD CHEM., UNIV. OF TURKU, FIN-20014 TURKU, FINLAND. TEL. +358 2 333 6894. FAX +358 2 333 6860. E-MAIL AOUWEHAN(A)BTK.UTU.FI, XP000952256

• SALJI J: "Acidophilus milk products: foods with a third dimension." FOOD SCIENCE & TECHNOLOGY TODAY 1992 45 PURBECK CLOSE, BEDFORD MK41 9LX, UK, vol. 6, no. 3, pages 142-147, XP000952420

• DATABASE FSTA [Online] INTERNATIONAL FOOD INFORMATION SERVICE (IFIS), FRANFURT/MAIN, DE; BOROVIC A ET AL: "Lactobacillus GG - probiotic property and activity." Database accession no. 1999-00-a0638 XP002150876 & MLJEKARSTVO 48 (3) 177-191 1998 MLJEKARA DUKAT' D.D. ZAGREB, ZAGREB, CROATIA,

• DATABASE WPI Section Ch, Week 199252 Derwent Publications Ltd., London, GB; Class D13, AN 1992-426647 XP002150878 & JP 04 320642 A (SNOW BRAND MILK PROD CO LTD), 11 November 1992 (1992-11-11)

• PERDIGON G ET AL: "PREVENTION OF GASTROINTESTINAL INFECTION USING IMMUNOBIOLOGICAL METHODS WITH MILK FERMENTED WITH LACTOBACILLUS CASEI AND LACTOBACILLUS ACIDOPHILUS" JOURNAL DAIRY RESEARCH,CAMBRIDGE,GB, vol. 57, 1990, pages 255-264, XP000916913

- **DATABASE BIOSIS [Online] BIOSCIENCES INFORMATION SERVICE, PHILADELPHIA, PA, US; 1997 KABAYAMA SHIGERU ET AL: "Enhancing effects of food components on the production of interferon beta from animal cells suppressed by stress hormones." Database accession no. PREV199799515042 XP002150877 & CYTOTECHNOLOGY, vol. 23, no. 1-3, 1997, pages 119-125, ISSN: 0920-9069**

**Description**

This invention relates to the use of lactic acid bacteria for the preparation of fermented milks intended for the prevention and treatment of temporarily reduced immune activity levels and normalizing immune activity levels that are depressed in comparison with what may be considered normal, especially in healthy individuals who are subject to intense exertion or in general to great physiological strain, caused directly by physical effort or induced by situations of psychological tension. In particular, it relates to the use of *Lactobacillus casei* in the preparation of fermented milk for alleviating the physiological depression of immunity levels in humans.

More specifically, this invention relates to means for improving the immune conditions of healthy individuals when they are subjected to different types of physiological stress.

The present invention has been developed as the result of investigations carried out by the inventors to find some preventive and/or therapeutic means to ensure a rapid recovery of a temporarily reduced immune activity, induced by physical or psychological stress. This invention has been arrived at following research into the effects of milks fermented with *Lactobacillus casei* in individuals engaging in vigorous exercise and, especially, athletes and, in general, individuals subjected to conditions of physiological stress. As is well known, there is growing interest in the specialist scientific community in the favourable effects produced by exercise. Nevertheless it has been observed that under conditions of very vigorous exercise such as in athletes, both during periods of training and during periods of competitive exercise, an inverse effect to that normally expected occurs in the immune system in the form of a reduction in the activity of the said immune system, and reports have been produced which describe mild infectious diseases in athletes as a result of the fall in their level of immunity after exhaustive exercise, as measured by a decrease in the number and activity of the cells known as NK ("natural killer") cells after intense and prolonged exercise.

As is well known, under the conditions of intense and prolonged exercise such as, for example, in athletes and other individuals engaging in very great effort, and, in general, individuals subjected to conditions of physiological stress, there has been a trend to help the individuals recover through isotonic drinks and/or vitamin drinks of various kinds.

Nevertheless, the inventors have observed that there have been no serious attempts in the scientific community to achieve a rapid restoration of immunity levels in individuals engaging in such intensive and prolonged exercise, with the result that in such persons there arises a situation in which, although natural restoration can permit the individual to recover the capacity to engage in major efforts after a period of exhaustion, levels of immune activity remain low for an extended period until natural recovery re-establishes them slowly. It is precisely the existence of these periods of low immune response in those individuals who are generally in a satisfactory state of health, as may be the case in athletes, which exposes them to the contraction of certain infections as a result of their temporary situation of having a low level of immune activity.

The inventors have carried out research and experiments specifically in order to overcome the disadvantage mentioned above, that is the temporarily low immune activity in individuals engaging in vigorous exercise. In the course of the investigations performed by the inventors, they have discovered, surprisingly, that it is possible to achieve, simultaneously, a less pronounced decrease in the level of immune activity and a more rapid restoration of said level in individuals in a generally satisfactory state of health, such as athletes engaging in intensive training and/or sporting trials, and, in general, individuals subjected to conditions of physiological stress, through administering fermented milks containing *Lactobacillus casei* to them. This surprising effect has been confirmed by the tests carried out and will be explained in the course of this description. The importance of this invention needs to be pointed out because of its wide-ranging effects and effectiveness and because it is not in line with the known use of fermented milks, with the special object of increasing calcium levels in the body, intestinal flora and other similar factors, whereas so far its use in persons in a generally satisfactory state of health, and even less so in the case of individuals who engage in intense and prolonged exercise has not been regarded as being of special interest.

It has been previously reported that, in patients exposed to pathogens, the absorption of fermented milk containing *Lactobacillus casei* reduces the severity of infections, particularly intestinal infections.

For example, Reinert et al. (Anales Espanoles de Pediatria, 1997) have demonstrated that the duration of acute diarrhoea in children was reduced by the consumption of fermented milk with *Lactobacillus casei*. However, no preventative effect was reported.

Elsewhere, it has been demonstrated that the ingestion of fermented milk products induces changes in the intestinal microflora and could exert an influence over digestive functions (Guerin-Danan et al., 1998).

Surprisingly, the inventors have now discovered that the ingestion of fermented milk containing *Lactobacillus casei* may contribute to a reduction in the level of depression of the systemic immune function in healthy individuals under conditions of physiological stress and also allow a more rapid restoration of normal levels, therefore achieving a preventative action, by limiting the temporary reduction of the immune level and accelerating the restoration of levels which existed prior to the situation of physiological stress.

Said physiological stress may occur, for example, after intense physical or intellectual activity, during pregnancy, as a result of psychological stress etc.

Therefore, one of the purposes of this invention is the use of orally administered fermented milks containing *Lactobacillus casei* for the purposes of achieving a significant effect on the restoration of immune activity; another purpose of the invention is the use of the fermented milks containing *Lactobacillus casei* for the prevention and reduction of the systemic immune function in healthy subjects in condition of physiological stress. It will therefore be particularly adapted in cases where there is a major temporary reduction in such levels when the individual is engaged in intense and prolonged physical activity.

The fermented milks contain at least one strain of *Lactobacillus casei*. In a preferred embodiment of the present invention, said strain is the strain deposited at the CNCM (Collection Nationale de Culture de Microorganismes, Institut Pasteur, 25 rue Docteur Roux) under number 1-1518 on 30 December 1994.

The fermented milks containing Lactobacillus casei may futher contain other lactic acid bacteria belonging to the genera *Lactobacillus, Lactococcus, Streptococcus* and *Bifidobacterium*. Said lactic acid bacteria are preferably chosen from a group comprising:

- *Lactobacillus helveticus*
- *Lactobacillus delbrueckii* subspecies *bulgaricus*
- *Lactobacillus paracasei*
- *Lactobacillus zeae*
- *Lactobacillus acidophilus*
- *Lactococcus lactis*
- *Streptococcus thermophilus*
- *Bifidobacterium longum and/or*
- *Bifidobacterium breve*

According to the invention, the fermented milk containing Lactobacillus casei may also contains a particular embodiment lactic acid bacteria, such as described in PCT application WO 96/20607.

In a preferred embodiment, the *Lactobacillus casei* strain will be present in the product in a minimum concentration of $10^5$ c.f.u./ml, more preferably between $10^6$ and $10^9$ c.f.u./ml. Products which provide good results according to the invention will contain *Lactobacillus casei* in concentrations in excess of $10^7$ c.f.u./ml.

*Lactobacillus casei* can be used for the preparation of fermented milks in a semi-solid or pasty form, similar to yoghurt, or in liquid form, for the treatment of temporary conditions characterized by a low level of immune activity.

The experiments were carried out on groups of athletes which showed a fall in the number of NK cells after intense effort.

## INDIVIDUALS AND METHODS

Individuals

Agreement to take part in the study was obtained from a group of 99 persons of both sexes. The individuals were committed athletes who engaged in physical aerobic activity at least three times per week for a period of more than 30 minutes, with an intensity of 60% of their maximum $VO_2$ or more. All the individuals were requested to abstain from taking the ergogenic aids which they were using for a minimum period of two months prior to starting the test. Individuals with known hypersensitivity or intolerance to milk products were excluded, as were individuals on special diets, such as weight reduction diets or vegetarian diets. Individuals who had experienced episodes of exercise-induced asthma and those who had received antibiotics or other medical treatment over a period up to one month prior to day -35 were also excluded.

Table 1 indicates the characteristics of the individuals engaged in the experiment.

Table 1

| Characteristics of individuals | | |
|---|---|---|
| | Mean | (Range) |
| Age (years) | 27 | (18-41) |
| Body mass index (kg/m$^2$) | 22.44 | (19.08-27.42) |
| Maximum $VO_2$ (kg/weight) | 43.39 | (33.6-77.9) |

Experimental design

The experiment comprised two parts. In the first part each individual engaged in a preset amount of cycloergometer exercise to achieve their maximum $VO_2$. After a 5 minute warm-up period with a generated power of 50 watts, the power was increased by 30 watts every 3 minutes until the level of the onset of tiredness was reached. Values of $VO_2$, heart rate, $pCO_2$, ventilation $pO_2$ and respiration quotient were measured every 30 seconds in exhaled air throughout the test. The EKG value was also measured continuously. Blood lactate concentration and the temperature of the tympanic membrane were also measured prior to the test and five minutes after it with the aim of evaluating the temperature in the central nervous system. Samples of venous blood were taken from the ear lobe for the analysis of lactate. The analysis was performed using a photoenzyme method measured using a Hitachi/Boehringer Mannheim 4020 photometer. The temperature of the tympanic membrane was measured using an infrared temperature scanner (Ototemp 3000 SD Natick, MA). After a rest for a period of between a week and 10 days, a second exertion test was performed on the cycloergometer at 75% of maximum $VO_2$ for 60 minutes. Before the maximum effort test case histories of the individuals and their families' health were obtained, together with a full blood analysis including blood count, cholesterol, HDL, LDL, glucose, uric acid, urea GOT, GPT and proteins.

The individuals attended the laboratory after a light breakfast (without milk or other milk products). After resting for some 20 minutes, each of the individuals engaged in exercise with a power of 50 watts for 5 minutes and subsequently at 75% of $VO_2$ for 60 minutes at 60 r.p.m. During the test ventilation, $O_2$ use ($VO_2$) and $CO_2$ emission ($VCO_2$) were monitored for periods of 30 minutes, 45 minutes and 60 minutes using Jaeger Bos-Sprint equipment (Erich Jaeger GmbH, Würzburg, Germany) calibrated before each of the tests. The equipment consisted of a paramagnetic oxygen analyser and of infrared equipment for the carbon dioxide ($CO_2$) and a Fleisch type pneumotachograph. Heart rate and EKG were likewise recorded continuously throughout the test. Blood was extracted for the analysis of NK cells and other parameters relating to immune level from the right antecubital vein with the individual lying down, before the test and again 5 minutes and 2 hours after the test. The concentration of lactates in blood and the temperature of the tympanic membrane were measured before the test and 5 minutes afterwards. The investigators believed that it would be useful to measure the temperature because it has been shown that hyperthermia can induce immune changes which are similar to the changes observed in relation to exercise (B.K. Pedersen et al., 1994, M.M. Hammami et al., 1998).

Second stage of the investigation

Twenty-five individuals were selected from a group of 94 (5 individuals did not wish to continue) for the second stage of the investigation. Selection was based on the fact that the individuals showed a significant reduction (more than 3%) in the number of NK cells in comparison with their basal plasma levels 2 hours after the test. During the second stage of the investigation the individuals were included in two parallel groups. Group 1 received 500 ml per day of orally administered milk fermented with *Lactobacillus casei* for a period of one month and Group 2 received 500 ml of orally administered milk per day for a period of one month. Fermented milk containing *Lactobacillus casei* was supplied at different intervals and was kept at a temperature of 2-6°C. At the end of each of the periods all the individuals were subjected to a 60 minute exercise test at 75% of their maximum $VO_2$ on the ergometer cycle at 60 r.p.m. Blood samples from the antecubital vein were obtained before the test and again at periods of 5 minutes and 2 hours after each test for an analysis of immune parameters.

The dietary history of each individual was obtained two days before the tests (after drinking milk fermented with *Lactobacillus casei* and milk) and analysed using the Professional Diet Balancer computer program (Nutridata Software Co., Wappingers Falls, NY, USA). This computer program has a database of 1600 food items with 23 nutrient values for each type of food. The program compares the total of each nutrient indicated by the individuals with the USA recommended daily amount (RDA) based on weight, age, sex and physical activity or energy consumption. It was useful to measure the percentage of carbohydrate in their total calorie intake. This is important because Nieman et al. (1997) have demonstrated that carbohydrates affect the redistribution of NK cells.

Fermented milk containing *Lactobacillus casei*

Milk fermented with *Lactobacillus casei* was prepared using traditional yoghurt cultures (L. bulgaricus and S. thermophilus) in addition to *Lactobacillus casei* (strain DN-114001). The presence of a minimum of $10^7$ colony forming units per ml (c.f.u./ml) of yoghurt bacteria was confirmed, with a content of $3.2 \times 10^8$ c.f.u./ml of *Lactobacillus casei* being found.

The composition of the nutrient was: 3.7 g of proteins, 3.3 g of fats and 5.2 g of carbohydrates per 100 g.

Methods

Sample collection and serum preparation

20 ml of venous blood were extracted from each athlete. 10 ml were collected in commercial heparinized tubes (Sarstedt, Germany) and 10 ml were allowed to coagulate for 30 minutes at ambient temperature in 10 ml tubes (Sarstedt, Germany). The tubes were centrifuged (1500 g) at ambient temperature and the serum was removed. All the samples were tested for immunoglobulins within the next 3 hours and they were then stored at -30°C before the cytokine test.

Blood cell counts

In the attached graphs:

Figure 1 shows the results with whole blood with CD3-FITC/CD16+CD56-PE staining and analysed on an Epics XL-MCL flow cytometer;
Figure 2 shows the temperature of the tympanic membrane measured by infrared thermography before and after the exercise test. The two lighter columns on the left of the graph show the temperatures with administration of milk, before and 5 minutes after, respectively, and the two darker graphs [sic] on the right show the situation corresponding to *Lactobacillus casei*;
Figure 3 shows the blood lactate concentration before and after an exercise test after the ingestion of fermented milk containing *Lactobacillus casei* and milk, the columns representing, respectively: before, 3 minutes after and 5 minutes after, the darker columns corresponding to fermented milk containing *Lactobacillus casei* and the lighter columns to milk;
Figure 4 shows a diagram of mean NKL values, the dark line corresponding to fermented milk with *Lactobacillus casei* and the light line to milk; and
Figure 5 shows, in a similar manner to Figure 4, the diagram of mean NKA values.

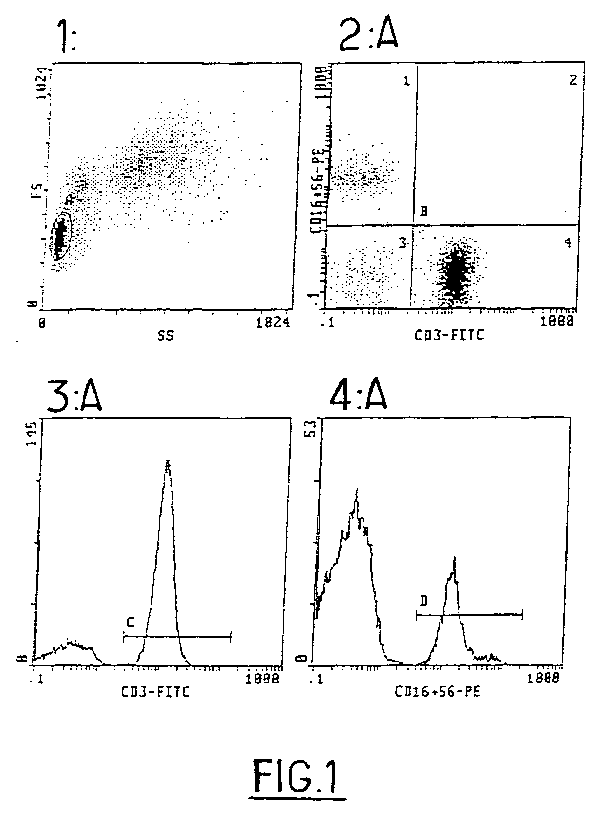

To determine the lymphocytes subsets, 100 µl of heparinized blood was mixed with 10 µl of selected monoclonal antibodies (mab) conjugated with fluoroscein isothiocyanate (FITC), phycoerythrin (PE) or Texas picoerythrin-red (ECD) in the following stain combinations: anti-CD4 mab (PE) / anti-CD8 mab (FITC) / anti-CD3 mab (ECD) from the Coulter Company (Coulter Corp. USA), anti-CD19 mab (PE) / anti-HLADR mab (FITC) / anti-CD3 mab (ECD), anti-CD14 mab (PE) / anti-CD64 mab (FITC) from the Immunotech company (Immunotech, France) and anti-CD16+CD56 mab (PE) / anti-CD3 mab (FITC) from the Becton Dickinson company (Becton Dickinson, USA) (Figure 1). After 15 minutes incubation in the dark, the samples were lysed with an Immunoprep solution in the Q-prep unit (Coulter Corp., USA). The lysate was immediately analysed using a Coulter Epics XL-MCL flow cytometer. The number of cells counted was 10,000 per sample. Findings were expressed as percentages of cells giving rise to specific fluorescence in a controlled lymphocytes region, with the exception of CD14/CD64 which was monitored in the monocytes region.

Activity of NK ("natural killer") cells

Mononuclear cells were isolated from peripheral blood and blood heparinized by centrifuging on a density gradient. 8 ml of whole blood was diluted 1:1 with PBS. This was placed in the form of layers on 5 ml of Lymphoprep (Nycomed, Norway) and centrifuged (600 g) for 20 minutes at 20°C. The layer of mononuclear cells was removed and washed twice with RPMI 1640 (GIBCO) supplemented with 10% of human AB serum (GIBCO). The pellet was reconstituted with a concentration of $2 \times 10^6$ cells/ml (effector cells).
NK activity was evaluated using the EuTDA cytotoxicity test (Wallac, Finland). 2 ml of K562 (ATCC-CCL 243) were added to $1 \times 10^6$ cells/ml and loaded with 5 µl of a ligand to encourage fluorescence (BATDA) at 20°C for 15 minutes. The cells were washed 6 times with PBS with 10% of BSA. Finally the pellet was adjusted with RPMI 1640 to $5 \times 10^4$ cells/ml (diana cells). 100 µl of these cells were placed in each well of a microtitration plate (Sarstedt, USA) containing effector cells to provide a diana: effector of 40:1, 20:1, 10:1 and 5:1. The microtitration plate was incubated at 37°C for 2 hours in an incubator with 5% of $CO_2$. When incubation was complete, the plate was centrifuged for 5 minutes at 500 g. Then 20 µl of the supernatant was transferred to a flat-bottomed plate (DELFIA microtitration plate); 200 µl of Eu solution were added to each of the wells and then incubated at ambient temperature for 15 minutes in a Sacudidas device. Finally fluorescence was measured in a DELFIA 1232 time-resolution fluorometer (Wallac). Controls included spontaneous release (100 µl of RPMI 1640 medium added to the labelled diana cells) and total release (100 µl of RPMI 1640 medium supplemented with 10% Tween, added to the labelled diana cells). Percentage lysis was calculated using the counts for each E:T ratio:

$$\%\text{lysis} = \frac{(\text{release from samples - spontaneous release})}{(\text{total release - spontaneous release})} \times 100$$

The results were standardized to lytic units (LU), calculated as the number of effector cells required for lysis of 20% of the $5 \times 10^3$ diana cells, and taken to be the number of lytic units included in $1 \times 10^7$ cells. The internal coefficient of variance for the test was 3%.

Immunoglobulins

Immunoglobulins (IgG, IgA and IgM) were quantitatively determined by nephelometry using BN II (Behring Nephelometer II) equipment. The reference curves were constructed by multi-point calibration based on IFCC standard CRM-470. In the protocol for the assay of sera the serum samples were automatically diluted and measured; the dilutions used were 1:20 (IgA and IgM) and 1:400 (IgG). The results are expressed in mg/dl.

Cytokines

Tests were made for IL-1beta, sIL-2R and IL-6 by chemoluminescent enzyme immunometric assay using an IMMULITE automatic analyser (Diagnostic Products Corp. DPC, USA). The detection limits were: 5 pg/ml for IL-1B, 10 U/ml for sIL-2R and 2 pg/ml for IL-6. IL-2 and INF-gamma were performed by Immunotech ELISA (Immunotech, France) in accordance with the manufacturer's instructions. The detection limits were 10 pg/ml for IL-2 and 0.5 U/ml for INF-gamma.

**RESULTS AND DISCUSSION**

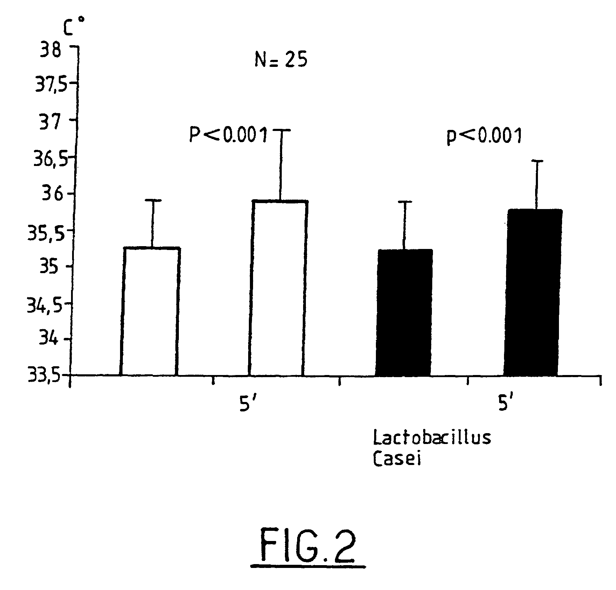

The exercise tests performed for treatment with milk and fermented milk showed the same level of increase in the temperature of the tympanum with respect to baseline values in 5 minutes (Figure 2). In no case did the temperature exceed 37°C. It has been shown that rectal temperature is 1°C higher than the tympanum temperature. It was shown by B.K. Pedersen et al. 1994 that a core temperature of 39°C can give rise to changes in the immune system which are similar to the changes experienced in connection with exercise. Thus no interference with the immune system after exercise could have been due to the increase in temperature observed in the investigation performed by the inventors.

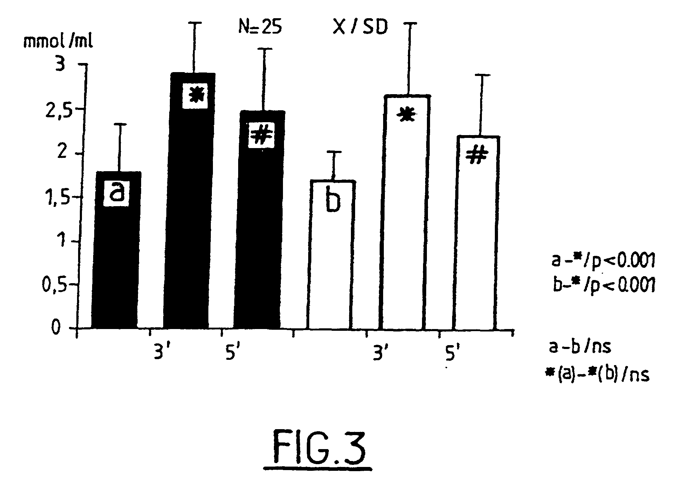

The baseline lactate concentration in blood and the increase at 3 and 5 minutes after exercise showed no significant difference between the two treatment programmes (Figure 3).

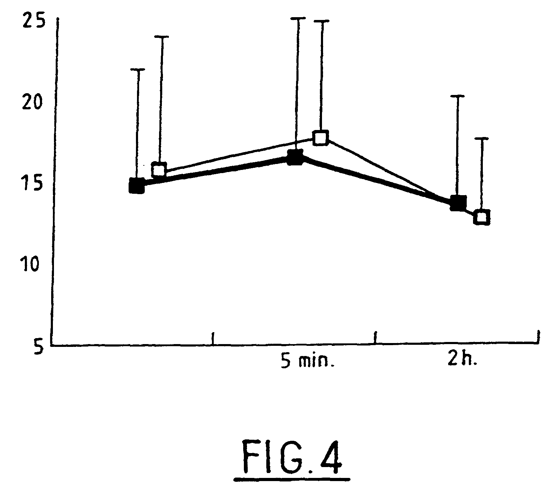

NK cells. - There was no difference in the concentration of NK cells in any of the points corresponding to the baseline 5 minutes and 2 hours after treatment between milk fermented with *Lactobacillus casei* and milk (Figure 2). A significant difference was observed after exercise in the response of the NK cell concentration between 5 minutes and 2 hours, both in the group having taken milk fermented containing *Lactobacillus casei* and the group having taken milk. The fall in NK cells observed after 2 hours was significantly less in the group having taken milk fermented with *Lactobacillus casei* (3.14 for milk fermented with *Lactobacillus casei* in comparison with 5.24 for milk; p<0.0035) (Figure 4).

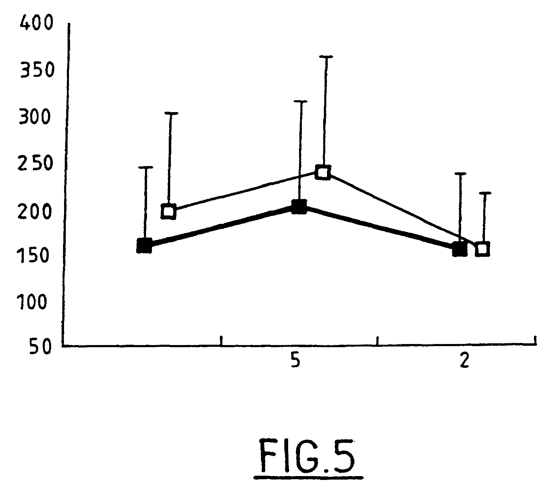

As far as the activity of the NK cells is concerned, a trend towards increased activity was observed in both groups (milk fermented with *Lactobacillus casei* and milk) between the base line and 5 minutes. A decrease between 5 minutes and 2 hours was also observed in the case of milk fermented with *Lactobacillus casei* and milk, the trend for milk fermented with *Lactobacillus casei* being less than that for milk (Figure 5).

The depressed immune function after periods of heavy exercise and excessive training is partly related to increased cortisone secretion and an acute protein stage mediated by IL-6 (J.A. Woods et al., 1999). The discovery that the ingestion of milk fermented with *Lactobacillus casei* can modulate the fall in the level of NK cells may partly help to counterbalance the immunosuppressive action of cortisone in athletes during intense training and competition.

According to the experiments done to ascertain the most efficacious effect of the new use for *Lactobacillus casei* in the preparation of fermented milks, the *Lactobacillus casei* content will be present in an amount in excess of $1 \times 10^7$ c.f.u. per millilitre.

All that which does not affect, alter, change or amend the essence of the new use described may be varied for the purposes of this invention.

## Claims

1. Use of *Lactobacillus casei* in the preparation of a fermented milk for the prevention and treatment of temporarily depressed immune levels associated with a decrease in blood NK cells in individuals subjected to physiological stress.

2. Use of claim 1, wherein said physiological stress results from intense physical effort.

3. Use of any of claims 1 or 2, wherein said fermented milk comprises more than $1 \times 10^5$ c.f.u. per millilitre of *Lactobacillus casei.*

4. Use of claim 3, wherein said fermented milk comprises more than $1 \times 10^7$ c.f.u. per millilitre of *Lactobacillus casei.*

5. Use of any of claims 1 to 4, wherein said *Lactobacillus casei* is strain CNCM I-1518.

6. Use of any of claims 1 to 5, wherein said fermented milk further comprises at least another lactic acid bacterium selected among *Lactobacillus helveticus, Lactobacillus delbrueckii* subspecies *bulgaricus, Lactobacillus paracasei, Lactobacillus acidophilus, Lactococcus lactis, Streptococcus thermophilus, Bifidobacterium longum* and/or *Bifidobacterium breve.*


## Patentansprüche

1. Verwendung von Lactobacillus casei bei der Herstellung von fermentierten Milchprodukten zur Prävention und Behandlung von temporär gesenkten Immunspiegeln, die mit einer Abnahme der Blut-NK-Zellen verbunden sind, bei Individuen, die physiologischem Stress ausgesetzt sind.

2. Verwendung nach Anspruch 1, wobei der physiologische Stress aus einer intensiven körperlichen Anstrengung resultiert.

3. Verwendung nach Anspruch 1 oder Anspruch 2, wobei die fermentierten Milchprodukte mehr als 1 x $10^5$ kbE pro Milliliter Lactobacillus casei umfassen.

4. Verwendung nach Anspruch 3, wobei die fermentierten Milchprodukte mehr als 1 x $10^7$ kbE pro Milliliter Lactobacillus casei umfassen.

5. Verwendung nach einem der Ansprüche 1 bis 4, wobei das Lactobacillus casei der Stamm CNCM I-1518 ist.

6. Verwendung nach einem der Ansprüche 1 bis 5, wobei die fermentierten Milchprodukte außerdem mindestens ein anderes Milchsäurebakterium, ausgewählt aus Lactobacillus helveticus, Lactobacillus delbrueckii Subspecies bulgaricus, Lactobacillus paracasei, Lactobacillus acidophilus, Lactococcus lactis, Streptococcus thermophilus, Bifidobacterium longum und/oder Bifidobacterium breve umfassen.


## Revendications

1. Utilisation de *Lactobacillus casei* pour la préparation d'un lait fermenté destiné à la prévention et au traitement d'un état **caractérisé par** des niveaux d'immunité temporairement déprimés, associés à une baisse du nombre de cellules NK dans le sang, chez des sujets soumis à un stress physiologique.

2. Utilisation selon la revendication 1, ledit stress physiologique résultant d'un effort physique intense.

3. Utilisation selon l'une quelconque des revendications 1 ou 2, ledit lait fermenté comprenant plus de 1 x $10^5$ UFC par millilitre de *Lactobacillus casei.*

4. Utilisation selon la revendication 3, ledit lait fermenté comprenant plus de 1 x $10^7$ UFC par millilitre de *Lactobacillus casei.*

**5.** Utilisation selon l'une quelconque des revendications 1 à 4, ledit *Lactobacillus casei* étant issu de la souche CNCM 1-1518.

**6.** Utilisation selon l'une quelconque des revendications 1 à 5, ledit lait fermenté comprenant, en outre au moins une autre bactérie lactique choisie parmi *Lactobacillus helveticus, Lactobacillus delbrueckii* sous-espèce *bulgaricus, Lactobacillus paracasei, Lactobacillus acidophilus, Lactococcus lactis, Streptococcus thermophilus, Bifidobacterium longum* et/ou *Bifidobacterium breve.*

FIG.1

FIG.2

FIG.3

## FIG.4

FIG.5